# FASCICULE DE BREVET EUROPEEN

(11) **EP 0 606 024 B1**
(45) Date de publication et mention de la délivrance du brevet: **20.03.1996**
(21) Numéro de dépôt: 93402703.8
(22) Date de dépôt: 04.11.1993
(51) Int. Cl.: C07H 17/08, A61K 31/70

(54) **Nouveaux dérivés de l'érythromycine, leur procédé de préparation et leur application comme médicaments**
Erythromycinderivate, ihr Verfahren zur Herstellung und ihre Verwendung als Arzneimitteln
Erythromycin derivatives, their process of preparation and their application as medicaments

(30) Priorité: 05.11.1992 FR 9213321
(43) Date de publication de la demande: 13.07.1994
(73) Titulaire: ROUSSEL UCLAF, F-93230 Romainville (FR)
(72) Inventeur: Bonnefoy, Alain, F-93260 Les Lilas (FR); Chantot, Jean-François, F-77410 Gressy en Françe (FR); Agouridas, Constantin, F-94130 Nogent sur Marne (FR); Denis, Alexis, F-75011 Paris (FR); Le Martret, Odile, F-75016 Paris (FR)
(74) Mandataire: Vieillefosse, Jean-Claude

(56) Documents cités:
- EP-A- 0 248 279
- EP-A- 0 487 411
- WO-A-92/09614

## Description

La présente invention concerne de nouveaux dérivés de l'érythromycine, leur procédé de préparation et leur application comme médicaments.

On connaissait déjà des dérivés de l'érythromycine, ne portant pas de cladinose en position 3, doués de propriétés antibiotiques (cf EP 0487 411).

L'invention a pour objet les composés de formule (I) : 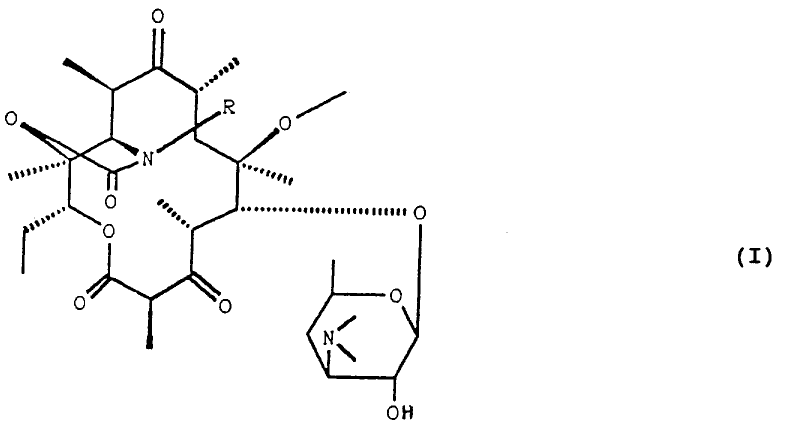 dans lesquels R représente un radical 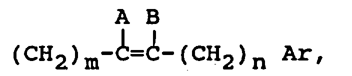 dans lequel m représente le nombre 1 ou 2 et n représente le nombre 0 ou 1
et ou bien A et B représentent chacun un atome d'hydrogène, la géométrie de la double liaison étant E ou Z ou un mélange E + Z,
ou bien A et B forment une troisième liaison entre les atomes de carbone auxquels ils sont liés,
et Ar représente :
- soit un radical aryle carbocyclique renfermant jusqu'à 18 atomes de carbone éventuellement substitué par un ou plusieurs radicaux choisis dans le groupe constitué par les radicaux carboxyle libre, salifié, estérifié ou amidifiés, le radical hydroxyle, les atomes d'halogène, les radicaux NO₂, C≡N, les radicaux alkyle, alkényle et alkynyle, O-alkyle, O-alkényle et O-alkynyle, S-alkyle, S-alkényle et S-alkynyle, N-alkyle, N-alkényle et N-alkynyle, linéaires, ramifiés ou cycliques renfermant jusqu'à 12 atomes de carbone éventuellement substitués par un ou plusieurs atomes d'halogènes, le radical 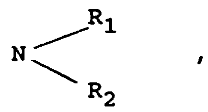 R₁ et R₂ identiques ou différents, représentant un atome d'hydrogène ou un radical alkyle renfermant jusqu'à 12 atomes de carbone, les radicaux aryle, 0-aryle et S-aryle hétérocycliques comportant un ou plusieurs hétéroatomes; éventuellement substitués par un ou plusieurs des substituants mentionnés ci-dessus,
ainsi que les sels d'addition avec les acides des composés de formule (I).

Dans la définition des substituants :
- le radical aryle carbocyclique est de préférence le radical phényle ou naphtyle,
- le radical alkyle, alkényle ou alkynyle est de préférence un radical méthyle, éthyle, propyle, isopropyle, n-butyle, isobutyle, terbutyle, décyle ou dodécyle, vinyle, allyle, éthynyle, propynyle, propargyle, cyclobutyle, cyclopentyle ou cyclohexyle,
- l'halogène est de préférence le fluor, le chlore ou le brome,
- le radical alkyle substitué par un atome d'halogène est de préférence un radical CHCl₂, CHBr₂, CHF₂, CCl₃, CBr₃, CF₃, CH₂CF₃, CH₂CH₂CCl₃, CH₂CH₂CF₃,
L'invention a plus particulièrement pour objet :
- les composés de formule (I) dans lesquels A et B représentent un atome d'hydrogène, ou forment une troisième liaison entre les carbones auxquels ils sont liés,
   les composés de formule (I) dans lesquels m représente le nombre 1 ou 2,
   les composés de formule (I) dans lesquels n représente le nombre 0 ou 1,
   les composés de formule (I) dans lesquels Ar représente un radical phényle éventuellement substitué par un atome de fluor, par un radical CF₃, par un radical phényle et tout spécialement ceux dans lesquels AR représente un radical phényle non substitué.

L'invention a tout spécialement pour objet le composé de l'exemple 1, et ses sels avec les acides.

Les produits de formule générale (I) possèdent une très bonne activité antibiotique sur les bactéries gram ^{⊕} telles que les staphylocoques, les streptocoques, les pneumocoques.

Les composés de l'invention peuvent donc être utilisés comme médicaments dans le traitement des infections à germes sensibles et notamment, dans celui des staphylococcies, telles que les septicémies à staphylocoques, staphylococcies malignes de la face ou cutanées, pyodermites, plaies septiques ou suppurantes, furoncles, anthrax, phlegmons, érysipèles et acné, staphylococcies telles que les angines aiguës primitives ou post-grippales, bronchopneumonies, suppuration pulmonaires, les streptococcies telles que les angines aiguës, les otites, les sinusites, la scarlatine, les pneumococcies telles que les pneumonies, les bronchites ; la brucellose, la diphtérie, la gonococcie.

Les produits de la présente invention sont également actifs contre les infections dues à des germes comme Haemophilus influenzae, Rickettsies, Mycoplasma pneumoniae, Chlamydia, Legionella, Ureaplasma, Toxoplasma, ou à des germes du genre Mycobactérium, Listeria, Meningocoques et Campylobacter.

La présente invention a donc également pour objet, à titre de médicaments et, notamment de médicaments antibiotiques, les produits de formule (I) tels que définis ci-dessus, ainsi que leurs sels d'addition avec les acides minéraux ou organiques pharmaceutiquement acceptables.

L'invention a plus particulièrement pour objet, à titre de médicaments et, notamment de médicaments antibiotiques, le produit de l'exemple 1 et ses sels pharmaceutiquement acceptables.

L'invention a également pour objet les compositions pharmaceutiques renfermant comme principe actif au moins un des médicaments définis ci-dessus.

Ces compositions peuvent être administrées par voie buccale, rectale, parentérale ou par voie locale en application topique sur la peau et les muqueuses, mais la voie d'administration préférée est la voie buccale.

Elles peuvent être solides ou liquides et se présenter sous les formes pharmaceutiques couramment utilisées en médecine humaine, comme par exemple, les comprimés, simples ou dragéifiés, les gélules, les granulés, les suppositoires, les préparations injectables, les pommades, les crèmes, les gels; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Ces compositions peuvent également se présenter sous forme d'une poudre destinée à être dissoute extemporanément dans un véhicule approprié, par exemple de l'eau stérile apyrogène.

La dose administrée est variable selon l'affection traitée, le sujet en cause, la voie d'administration et le produit considéré. Elle peut être, par exemple, comprise entre 50 mg et 300 mg par jour par voie orale, chez l'adulte pour le produit de l'exemple 1.

L'invention a également pour objet un procédé de préparation des composés de formule (I) caractérisé en ce que l'on soumet un composé de formule (II) : 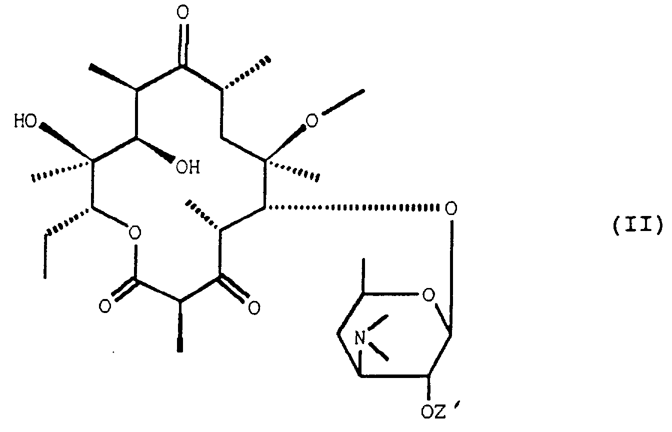 dans laquelle Z' représente le reste d'un acide carboxylique renfermant jusqu'à 18 atomes de carbone, à l'action d'un agent capable d'activer sélectivement l'hydroxyle en 11 pour obtenir le composé de formule (III) : 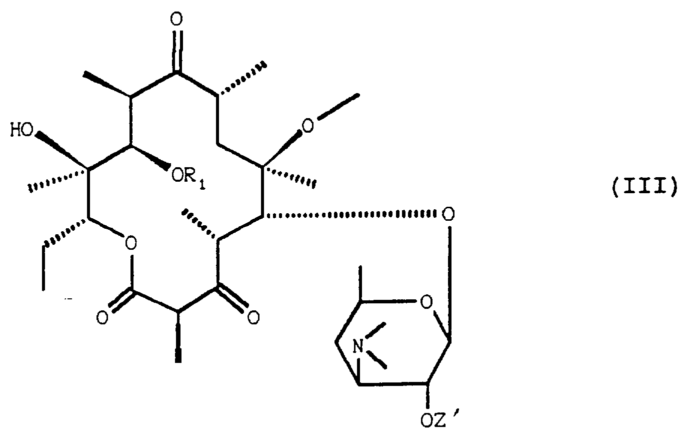 dans laquelle R₁ représente le reste du groupement activateur, que l'on soumet à l'action d'une base, pour obtenir le composé de formule (IV) : 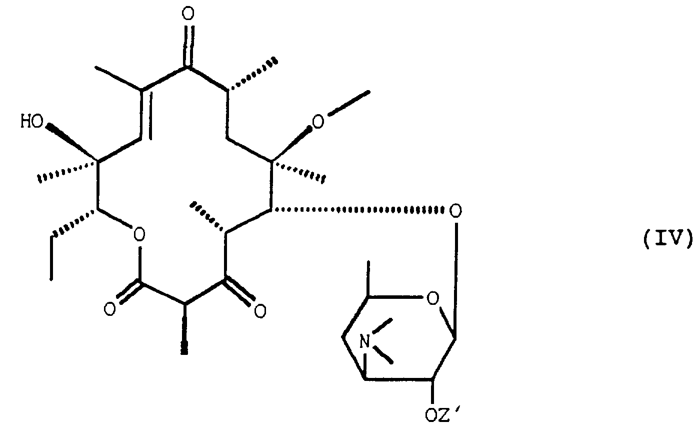 puis soumet le composé de formule (IV) :
- soit à l'action d'un composé de formule (V) :

   R-N=C=O (V)

   dans laquelle R a la signification définie précédemment, pour obtenir le composé de formule (VI) : 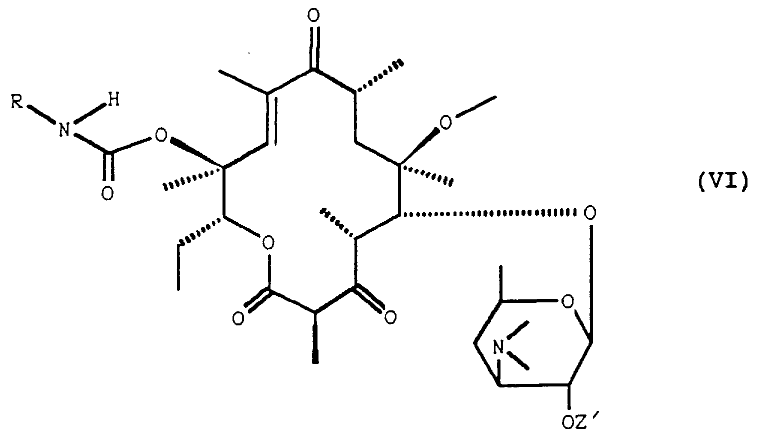 qui se cyclise soit spontanément par chauffage, soit que l'on soumet à l'action d'un agent de cyclisation pour obtenir le composé de formule (IA) : 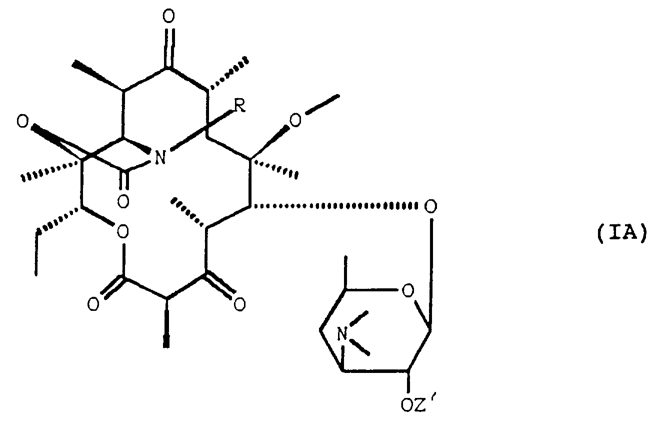 correspondant au produit de formule (I) dans laquelle Z ne représente pas un atome d'hydrogène,
- soit à l'action du carbonyldiimidazole pour obtenir le composé de formule (VII) : 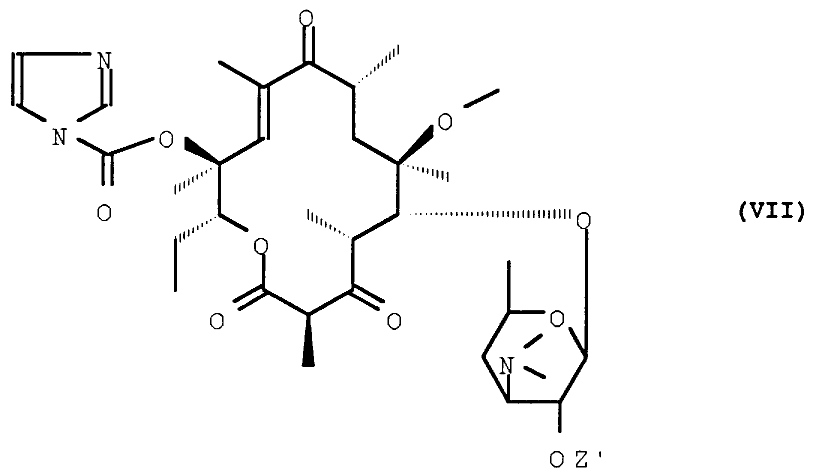 puis à l'action d'un composé de formule (VIII) :

   RNH₂ (VIII)

   R étant défini comme précédemment, pour obtenir le composé de formule (VI) défini précédemment qui se cyclise spontanément par chauffage ou que l'on soumet à l'action d'un agent de cyclisation pour obtenir le composé de formule (IA) correspondant, puis soumet, le composé de formule (IA) à l'action d'un agent de libération de la fonction hydroxyle en 2' et/ou le cas échéant, à l'action d'un acide pour en former le sel.

Dans un mode de réalisation préféré du procédé de l'invention :
- l'agent capable d'activer sélectivement l'hydroxyle en 11 est un anhydride sulfonique tel que l'anhydride méthane sulfonique, paratoluène sulfonique ou trifluorométhane sulfonique,
- la base capable de créer une double liaison 10(11) est un diazabicycloundécène par exemple le DBU (ou 1,8 diazabicyclo-[5-4-0]undec-7-ène), ou le diazabicyclononène, ou la 2,6-lutidine, ou la 2,4,6-collidine ou la tétraméthylguanidine,
- la réaction entre le composé de formule (IV) et le composé de formule (V) a lieu en présence d'une base, comme la pyridine, la triéthylamine, la morpholine, la N-méthyl morpyridine, la triéthylamine, la morpholine, la N-méthyl morpholine, la cyclisation du composé de formule (VI) intervenant soit spontanément soit par chauffage à une température comprise entre 50° et 100°,
- la réaction du composé de formule (IV) avec le carbonyldiimidazole a lieu en présence d'une base comme l'hydrure de sodium, ou la triéthylamine, ou un carbonate ou un carbonate acide de sodium ou de potassium, ou en l'absence de base dans un solvant tel que le chlorure de méthylène, le tétrahydrofuranne ou le diméthylformamide,
- la réaction du composé de formule (VII) avec le composé RNH₂ a lieu au sein d'un solvant tel que par exemple l'acétonitrile, le diméthylformamide ou encore le tétrahydrofuranne, le diméthoxy éthane ou le diméthylsulfoxyde, la cyclisation du composé de formule (VI) se produisant en général au cours de la réaction ou intervenant par action, sur le composé de formule (VI) isolé, d'une base telle que le terbutylate de potassium, au sein d'un solvant tel que le tétrahydrofuranne,
- l'hydrolyse de la fonction ester en 2' est réalisée à l'aide du méthanol ou de l'acide chlorhydrique aqueux,
- la salification est réalisée au moyen d'acides selon les procédés classiques.

Les composés de formule (II) utilisés comme produits de départ sont des produits connus d'une façon générale et peuvent être préparés comme il est indiqué dans la demande de brevet européen 0 487 411.

Les composés de formule RN=C=O et RNH₂ sont des produits connus d'une façon générale.

Les composés de formule RNH₂ peuvent par exemple être préparés selon les procédés décrits dans Ann. Chem. 690-98-114.

Les exemples suivants illustrent l'invention sans toutefois la limiter.

### EXEMPLE 1 : 11,12-didéoxy 3-de((2,6-didéoxy 3-C-méthyl 3-O-méthyl alpha-L-ribohexopyranosyl) oxy) 6-O-méthyl 3-oxo 12,11-(oxycarbonyl ((4-phényl 3-butényl) imino)) érythromycine

### STADE A : 2'-acétate de 3-de((2,6-didéoxy 3-C-méthyl 3-O-méthyl alpha-L-ribohexopyranosyl) oxy) 6-O-méthyl 11-O-(méthylsulfonyl) 3-oxo érythromycine

On introduit sous agitation et sous atmosphère d'azote 17 g de 2'-acétate de 3-de((2,6-didéoxy 3-C-méthyl 3-O-méthyl alpha-L-ribohexopyranosyl) oxy) 6-O-méthyl 3-oxoérythromycine dans 100 ml de pyridine. On refroidit le mélange obtenu jusqu'à 10°C. On ajoute 11,9 g d'anhydride méthane sulfonique. On laisse revenir à la température ambiante. On maintient l'agitation pendant 5 heures. On filtre le précipité obtenu. On concentre, reprend à l'eau et extrait à l'acétate d'éthyle. On lave à l'eau les phases organiques, les sèche, filtre et concentre. On obtient ainsi 20,9 g de produit recherché brut que l'on purifie par salification à l'aide d'acide oxalique puis libération de la base avec de l'ammoniaque. On obtient ainsi 15,16 g du produit recherché (F = 210∼212°C)

### STADE B : 2'-acétate de 11-déoxy 10,11-didéhydro 3-de(2,6-didéoxy 3-C-méthyl 3-O-méthyl alpha-L-ribohexopyranosyl) oxy) 6-O-méthyl 3-oxo érythromycine

On introduit sous agitation 8,26 g de produit préparé au stade A dans 35 ml d'acétone. On ajoute ensuite goutte à goutte 2,19 ml de DBU. On maintient l'agitation à la température ambiante pendant 20 heures. On reprend le mélange réactionnel avec du chlorure de méthylène. On lave à l'eau les phases organiques, les sèche sur sulfate de sodium, filtre et concentre. On obtient 10 g de produit que l'on reprend dans l'éther. On essore et lave à l'éther éthylique. On obtient ainsi 6,33 g de produit fondant à 230∼232°C.

### STADE C : 2'-acétate de 11-déoxy 10,11-didéhydro 3-de(2,6-didéoxy 3-C-méthyl 3-O-méthyl alpha-L-ribohexopyranosyl) oxy) 12-O-((1H-imidazol-1-yl) carbonyl) 6-O-méthyl 3-oxo érythromycine

On introduit 96 mg d'hydrure de sodium à 50 % dans l'huile dans 15 ml de tétrahydrofuranne. On refroidit la suspension obtenue à 0°C et introduit goutte à goutte une solution de 611 mg de produit préparé au stade précédent dans 17 ml de tétrahydrofuranne. On introduit à 0°C une solution de 486 mg de carbonyldiimidazole dans 15 ml de tétrahydrofuranne. furanne. On maintient l'agitation pendant 4 h 30. On laisse le mélange réactionnel revenir à la température ambiante, filtre et concentre. On reprend à l'acétate d'éthyle, lave au dihydrogénophosphate de sodium, extrait à l'acétate d'éthyle, sèche, filtre et concentre. On obtient 852 mg de produit recherché.

### STADE D : 2'-acétate de 11,12-didéoxy 3-de((2,6-didéoxy 3-C-méthyl 3-O-méthyl alpha-L-ribohexopyranosyl) oxy) 6-O-méthyl 3-oxo 12,11-(oxycarbonyl ((4-phényl 3-butényl) imino)) érythromycine

On agite pendant 5 h 30 à 55°C, un mélange de 0,9 g de 2'-acétate de 11-déoxy 10,11-didéhydro 3-de((2,6-didéoxy 3-C-méthyl 3-O-méthyl alpha-L-ribohexopyranosyl) oxy) 12-O-((1H-imidazol 1-yl) carbonyl) 6-O-méthyl 3-oxo érythromycine, 3 ml d'acétonitrile et 0,6 g de 4-phényl 3-buténylamine. On verse le mélange réactionnel sur une solution aqueuse 0,5 M de dihydrogénophosphate de sodium, extrait à l'acétate d'éthyle, lave à l'eau, sèche, filtre et concentre. On obtient 0,9 g d'huile que l'on chromatographie sur silice, en éluant avec le mélange acétate d'éthyle-triéthylamine (96-4). On rassemble les phases homogènes en CCM et concentre. On obtient 0,32 g d'un produit que l'on empâte dans l'hexane, essore et sèche à 70°C. On isole 0,215 g de produit fondant à 201-203°C.

### STADE E : 11,12-didéoxy 3-de((2,6-didéoxy 3-C-méthyl 3-O-méthyl alpha-L-ribohexopyranosyl) oxy) 6-O-méthyl 3-oxo 12,11-(oxycarbonyl ((4-phényl 3-butényl) imino)) érythromycine

On maintient sous agitation à la température ambiante pendant 15 heures, une solution renfermant 0,194 g de produit préparé au stade A et 6 ml de méthanol. On évapore le méthanol au rotorvapor. On chromatographie le produit obtenu sur silice en éluant avec le mélange chlorure de méthylène-méthanol-ammoniaque (95-5-0,2). On rassemble les phases homogènes, les concentre, les sèche, les filtre et concentre. On obtient 0,155 g de produit que l'on essore et sèche à 70°C. On obtient 0,124 g de produit fondant à 257∼259°C.

En opérant comme à l'exemple 1 en utilisant les amines appropriées, on a préparé les produits suivants :

### EXEMPLE 2 : (Z) 11,12-didéoxy 3-de((2,6-didéoxy 3-C-méthyl 3-O-méthyl alpha-L-ribohexopyranosyl) oxy) 6-O-méthyl 3-oxo 12,11-(oxycarbonyl ((4-(4-fluorophényl 3-butényl) imino)) érythromycine

F = 222°C.

### EXEMPLE 3 : (Z) 11,12-didéoxy 3-de((2,6-didéoxy 3-C-méthyl 3-O-méthyl alpha-L-ribohexopyranosyl) oxy) 6-O-méthyl 3-oxo 12,11-(oxycarbonyl ((4-(4-trifluorométhyl 3-butényl) imino)) érythromycine

F = 230°C.

### EXEMPLE 4 : (cis) 11,12-didéoxy 3-de((2,6-didéoxy 3-C-méthyl 3-O-méthyl alpha-L-ribohexopyranosyl) oxy) 6-O-méthyl 3-oxo 12,11-(oxycarbonyl ((4-phényl 3-butényl) imino)) érythromycine

F = 220°-225°C.
[α]_{D} = + 10,5° (c = 0,9% CHCl₃)

### EXEMPLE 5 : (E) 11,12-didéoxy 3-de((2,6-didéoxy 3-C-méthyl 3-O-méthyl alpha-L-ribohexopyranosyl) oxy) 6-O-méthyl 3-oxo 12,11-(oxycarbonyl ((4-phényl 2-butényl) imino)) érythromycine

F ≈ 78°C.

### EXEMPLE 6 : (Z) 11,12-didéoxy 3-de((2,6-didéoxy 3-C-méthyl 3-O-méthyl alpha-L-ribohexopyranosyl) oxy) 6-O-méthyl 3-oxo 12,11-(oxycarbonyl ((4-phényl 3-butényl) imino)) érythromycine

F = 220°C.
[α]_{D} = + 16° (c = 1% CHCl₃)

### EXEMPLE 7 : 11,12-didéoxy 3-de((2,6-didéoxy 3-C-méthyl 3-O-méthyl alpha-L-ribohexopyranosyl) oxy) 6-O-méthyl 3-oxo 12,11-(oxycarbonyl ((4-phényl 3-butynyl) imino)) érythromycine

F = 252°C.

### EXEMPLE 8 : 11,12-didéoxy 3-de((2,6-didéoxy 3-C-méthyl 3-O-méthyl alpha-L-ribohexopyranosyl) oxy) 6-O-méthyl 3-oxo 12,11-(oxycarbonyl ((4-phényl 2-butynyl) imino)) érythromycine

F ≈ 98°C.

### EXEMPLE 9 : (E) 11,12-didéoxy 3-de((2,6-didéoxy 3-C-méthyl 3-O-méthyl alpha-L-ribohexopyranosyl) oxy) 6-O-méthyl 3-oxo 12,11-(oxycarbonyl ((4-(1,2'-biphényl 4-yl) 3-butényl) imino)) érythromycine

F = 215-217°C.
[α]_{D} = - 46,5° (c = 1% CHCl₃)

### EXEMPLE 10 : (Z) 11,12-didéoxy 3-de((2,6-didéoxy 3-C-méthyl 3-O-méthyl alpha-L-ribohexopyranosyl) oxy) 6-O-méthyl 3-oxo 12,11-(oxycarbonyl ((4-(1,2'-biphényl 4-yl) 3-butényl) imino)) érythromycine

F = 133-137°C.
[α]_{D} = - 2,5° (c = 1% CHCl₃)

En opérant de manière identique, on a préparé les produits suivants :

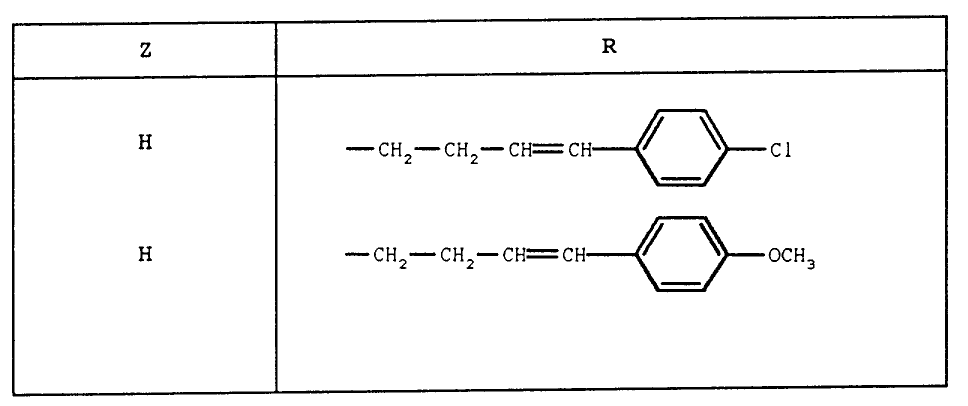

### PREPARATION 1 : [4-fluorophényl-4-yl] 3-buténylamine

### STADE A : N-[[4-fluorophényl-4-yl] 3-butényl] phtalimide.

On refroidit à -40°C, une suspension renfermant 150 ml de tétrahydrofuranne, 6 g de 4-fluorobenzaldéhyde et 25,6 g de bromure de triphényl phosphonium du N-(3-bromopropyl) phtalimide. On introduit ensuite 5,42 g de terbutylate de potassium. On laisse la température remonter jusqu'à -15°C et maintient l'agitation pendant 1 heure.

On verse sur de la glace, extrait à l'acétate d'éthyle, lave à l'eau, sèche les phases organiques sur Na₂SO₄, filtre et concentre. On obtient 26,7 g de produit que l'on dissout dans le chlorure de méthylène et chromatographie sur silice en éluant avec le mélange acétate d'éthyle-hexane (1-9). On concentre sous pression réduite et isole 5,2 g de produit recherché fondant à 94°C.

### Microanalyse :

| | % calculé | % trouvé | |
|---|---|---|---|
| C % | 73,40 | C % | 73,2 |
| H % | 4,77 | H % | 4,8 |
| N % | 4,74 | N % | 4,6 |
| F % | 4,43 | F % | 6,6 |

### STADE B : [4-fluorophényl-4-yl] 3-buténylamine

On agite 16 heures à 0°C un mélange renfermant 200 ml d'éthanol, 5,5 g de produit préparé comme au stade A, et 1,5 ml d'hydrate d'hydrazine. On laisse revenir à la température ambiante, évapore le solvant, reprend le résidu à l'éther, acidifie jusqu'à pH = 1 à l'aide d'une solution d'acide chlorhydrique 2N, élimine le solvant, reprend à l'eau, ajoute une solution de carbonate de sodium, extrait à l'éther et évapore les solvants. Après chromatographie du résidu sur silice (éluant : chlorure de méthylène-méthanol-ammoniaque 9-1-0,5), on obtient 2,2 g de produit attendu.

### PREPARATION 2 : [4-trifluorométhyl phényl-4-yl] 3-buténylamine

### STADE A : N-[[4-trifluorométhyl-4-yl] 3-butényl] phtalimide.

On opère comme au stade A de la préparation 1 en utilisant au départ 6 g de 4-(trifluorométhyl) benzaldéhyde. On obtient 4 g de produit attendu. F = 88°C.

### STADE B : [4-trifluorométhyl phényl-4-yl] 3-buténylamine

On opère comme au stade B de la préparation 1 en utilisant au départ 2,13 g de produit obtenu au stade A et 0,84 cm³ d'hydrate d'hydrazine. On obtient 0,850 g de l'amine attendue.

### PREPARATION 3 : 4-[(1,1'-biphényl) 4-yl] 3-buténylamine

### STADE A : N-[4-[(1,1'-biphényl) 4-yl] 3-butényl] phtalimide.

On refroidit à -40°C, une suspension renfermant 150 ml de tétrahydrofuranne, 5,46 g de 4-phénylbenzaldéhyde et 15,9 g de bromure triphényl phosphonium du N-(3-bromopropyl) phtalimide. On introduit ensuite 3,37 g de terbutylate de potassium. On laisse la température remonter jusqu'à -15°C et maintient l'agitation à -15°C pendant 1 heure.

On verse sur de la glace, extrait à l'acétate d'éthyle, lave à l'eau, sèche les phases organiques sur Na₂SO₄, filtre et concentre. On obtient 19 g de produit que l'on dissout dans le chlorure de méthylène et chromatographie sur silice en éluant avec le mélange acétate d'éthyle-hexane (3-7). On concentre. On empâte dans l'hexane, on essore, on sèche sous pression réduite et isole 8,5 g de produit recherché fondant à 112 ∼ 114°C.

### Microanalyse :

| | % calculé | % trouvé | |
|---|---|---|---|
| C % | 81,56 | C % | 81,4 |
| H % | 5,42 | H % | 5,3 |
| N % | 3,96 | N % | 3,8 |

### STADE B : 4-[(1,1'-biphényl) 4-yl] 3-buténylamine

On porte au reflux un mélange renfermant 280 ml d'éthanol, 7,9 g de produit préparé au stade A, et 1,3 ml d'hydrate d'hydrazine. On laisse revenir à la température ambiante, filtre le précipité obtenu et le lave à l'éthanol. On concentre, verse sur une solution d'acide chlorhydrique 2N et extrait à l'acétate d'éthyle. On lave à l'eau les phases organiques, les sèche, filtre et concentre sous pression réduite. On obtient 2,89 g de produit recherché.
F = 188 ∼ 194°C.

### EXEMPLE DE COMPOSITION PHARMACEUTIOUE

On a préparé des comprimés renfermant :

| | |
|---|---|
| **Produit de l'exemple 1** | 150 mg |
| Excipient q.s.p. | 1 g |

Détail de l'excipient : amidon, talc, stéarate de magnésium

### ETUDE PHARMACOLOGIQUE DES PRODUITS DE L'INVENTION

### Méthode des dilutions en milieu liquide

On prépare une série de tubes dans lesquels on répartit une même quantité de milieu nutritif stérile. On distribue dans chaque tube des quantités croissantes du produit à étudier, puis chaque tube est ensemencé avec une souche bactérienne.

Après incubation de vingt-quatre heures à l'étuve à 37°C, l'inhibition de la croissance est appréciée par transillumination de ce qui permet de déterminer les concentrations minimales inhibitrices (C.M.I.) exprimées en microgrammes/cm³.

Les résultats suivants ont été obtenus avec le produit de l'exemple 1 : (lecture après 24 heures)

### Souches bactériennes à GRAM⁺

| | |
|---|---|
| Staphylococcus aureus 011UC4 | 0,04 |
| Staphylococcus aureus 011HT17 | 0,04 |
| Staphylococcus aureus 011G025I | 0,08 |
| Staphylococcus epidermidis 012GO11C | 0,08 |
| Streptococcus pyogenes | ≤ 0,02 |
| groupe A 02A1UC1 | |
| Streptococcus agalactiae | 0,08 |
| groupe B 02B1HT1 | |
| Streptococcus sp | ≤ 0,02 |
| groupe C 02C0CB3 | |
| Streptococcus faecalis | 0,04 |
| groupe D 02D2UC1 | |
| Streptococcus faecium | ≤ 0,02 |
| groupe D 02D3HT1 | |
| Streptococcus sp | ≤ 0,02 |
| groupe G 02G0GR5 | |
| Streptococcus mitis | ≤ 0,02 |
| 02mitCB1 | |
| Streptococcus agalactiae | 0,3 |
| groupe B 02B1SJ1 | |
| Streptococcus sp | 0,15 |
| groupe C 02C0CB1 | |
| Streptococcus pneumoniae | 0,08 |
| 032UC1 | |
| Streptococcus pneumoniae | 2,5 |
| 030SJ1 | |
| Streptococcus pneumoniae | 0,08 |
| 030SJ5 | |

De plus, le produit de l'exemple 1, a manifesté une activité intéressante sur les souches bactériennes à GRAM⁻ Haemophilus Influenzae 351HT3, 351CB12, 351CA1 et 351GR6.

## Revendications

1. Les composés de formule (I) : 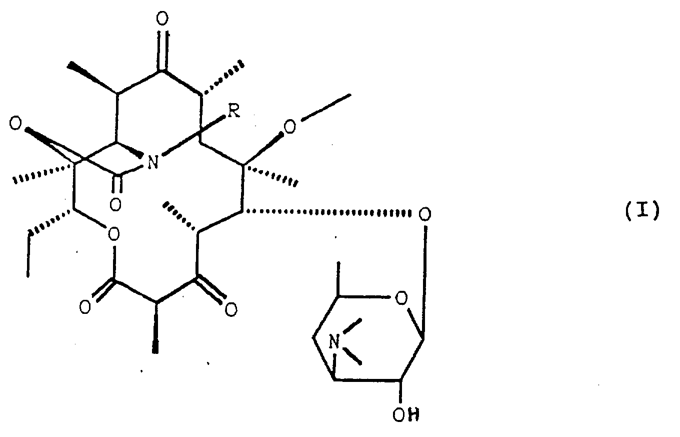 dans lesquels R représente un radical 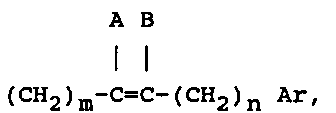 dans lequel m représente le nombre 1 ou 2 et n représente le nombre 0 ou 1,
et ou bien A et B représentent chacun un atome d'hydrogène, la géométrie de la double liaison étant E ou Z ou un mélange E + Z,
ou bien A et B forment une troisième liaison entre les atomes de carbone auxquels ils sont liés,
et Ar représente un radical aryle carbocyclique renfermant jusqu'à 18 atomes de carbone éventuellement substitué par un ou plusieurs radicaux choisis dans le groupe constitué par les radicaux carboxyle libre, salifié, estérifié ou amidifié, le radical hydroxyle, les atomes d'halogène, les radicaux NO₂, C≡N, les radicaux alkyle, alkényle et alkynyle, O-alkyle, O-alkényle et O-alkynyle, S-alkyle, S-alkényle et S-alkynyle, N-alkyle, N-alkényle et N-alkynyle, linéaire, ramifiés ou cycliques renfermant jusqu'à 12 atomes de carbone, éventuellement substitués par un ou plusieurs atomes d'halogènes, le radical 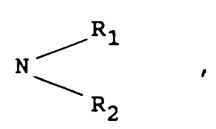 R₁ et R₂ identiques ou différents, représentant un atome d'hydrogène ou un radical alkyle renfermant jusqu'à 12 atomes de carbone, les radicaux aryle, O-aryle et S-aryle hétérocycliques comportant un ou plusieurs hétéroatomes, éventuellement substitués par un ou plusieurs des substituants mentionnés ci-dessus, ainsi que leurs sels d'addition avec les acides.

2. Les composés de formule (I) tels que définis à la revendication 1, dans lesquels Ar représente un radical phényle éventuellement substitué par un atome de fluor, un radical CF₃, un radical phényle.

3. Les composés de formule (I) tels que définis à la revendication 2, dans laquelle Ar représente un radical phényle.

4. Le composé de formule (I) défini à la revendication 1 dont le nom suit :
- 11,12-didéoxy 3-de((2,6-didéoxy 3-C-méthyl 3-O-méthyl alpha-L-ribohexopyranosyl) oxy) 6-O-méthyl 3-oxo 12,11-(oxycarbonyl ((4-phényl 3-butényl) imino)) érythromycine, et ses sels avec les acides.

5. A titre de médicaments les composés de formule (I) tels que définis à l'une quelconque des revendications 1, 2 et 3 ainsi que les sels d'addition avec les acides pharmaceutiquement acceptables.

6. A titre de médicament le composé de formule (I) tel que défini à la revendication 4, ainsi que ses sels d'addition avec les acides pharmaceutiquement acceptables.

7. Les compositions pharmaceutiques renfermant comme principe actif au moins un médicament défini à la revendication 5 ou 6.

8. Procédé de préparation des composés de formule (I) telle que définie à l'une quelconque des revendications 1 à 4, caractérisé en ce que l'on soumet un composé de formule (II): 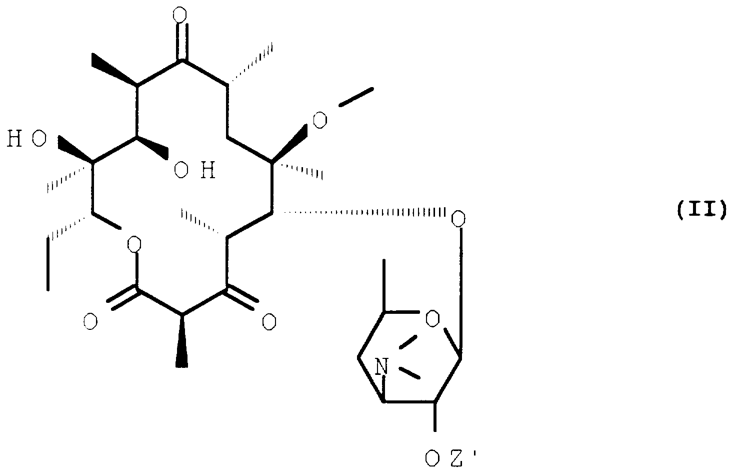 dans laquelle Z' représente le reste d'un acide carboxylique renfermant jusqu'à 18 atomes de carbone, à l'action d'un agent capable d'activer sélectivement l'hydroxyle en 11 pour obtenir le composé de formule (III) : 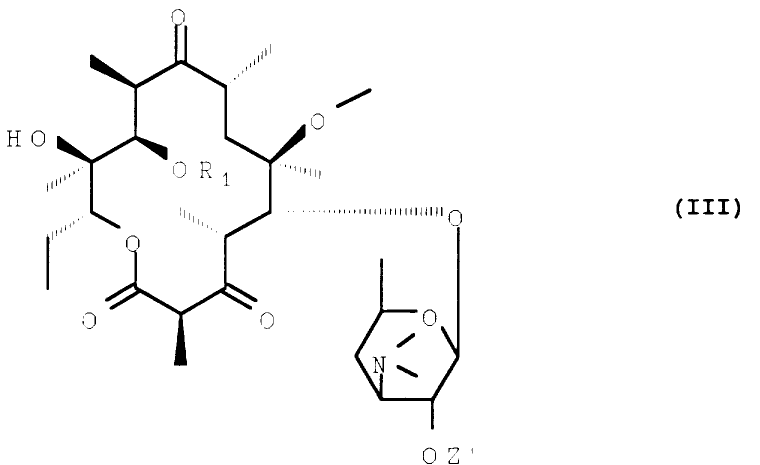 dans laquelle R₁ représente le reste du groupement activateur, que l'on soumet à l'action d'une base, pour obtenir le composé de formule (IV) : 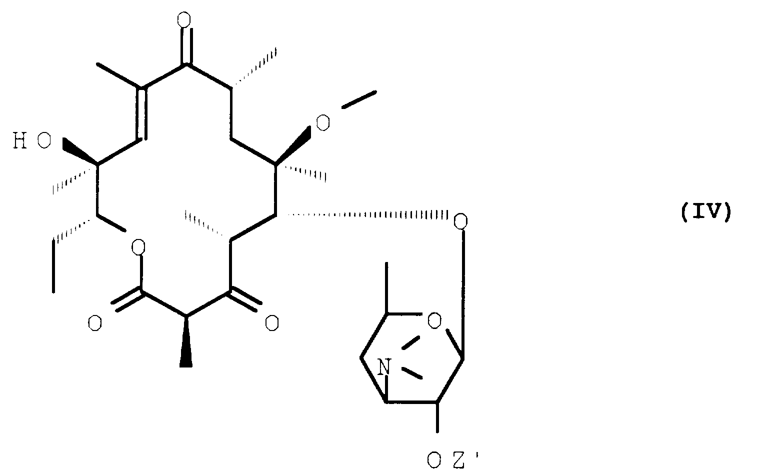 puis soumet le composé de formule (IV) :
- soit à l'action d'un composé de formule (V) :
R-N=C=O (V)
dans laquelle R est défini comme à la revendication 1, pour obtenir le composé de formule (VI) : 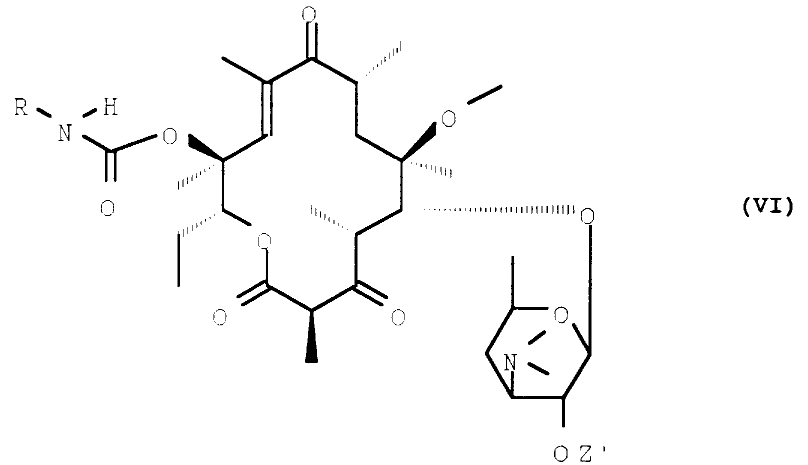 qui se cyclise soit spontanément par chauffage, soit que l'on soumet à l'action d'un agent de cyclisation pour obtenir le composé de formule (IA) : 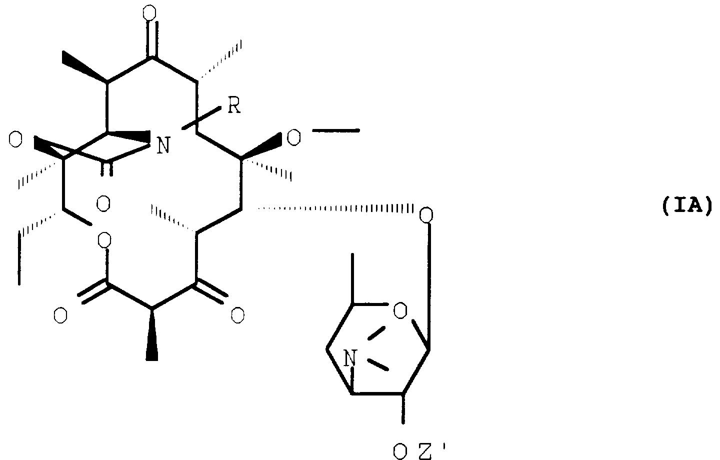
- soit à l'action du carbonyldiimidazole pour obtenir le composé de formule (VII) : 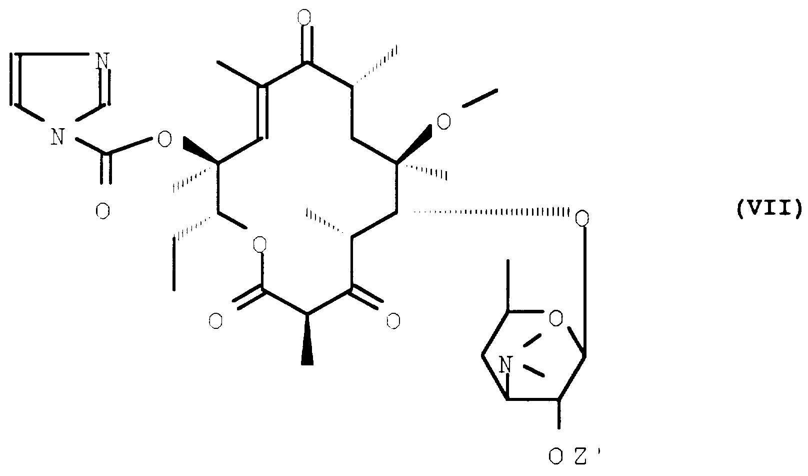 puis à l'action d'un composé de formule (VIII) :
RNH₂ (VIII)
dans laquelle R est défini comme à la revendication 1 pour obtenir le composé de formule (VI) défini précédemment qui se cyclise spontanément par chauffage ou que l'on soumet à l'action d'un agent de cyclisation pour obtenir le composé de formule (IA) correspondant, puis soumet le composé de formule (IA) à l'action d'un agent de libération de la fonction hydroxyle en 2' et/ou le cas échéant, à l'action d'un acide pour en former le sel.

## Patentansprüche

1. Verbindungen der Formel (I) 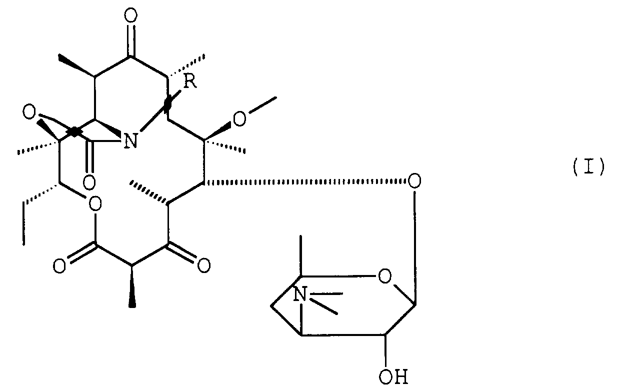 in der R einen Rest 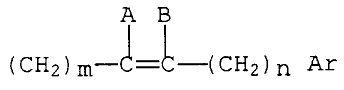 darstellt, worin m eine Zahl von 1 oder 2 und n die Zahl 0 oder 1 bedeuten,
und A und B entweder jeweils ein Wasserstoffatom darstellen, wobei die Geometrie der Doppelbindung E oder Z oder eine Mischung von E + Z ist,
oder A und B eine dritte Bindung zwischen den Kohlenstoffatomen bilden, an die sie gebunden sind,
und Ar einen carbocyclischen Arylrest mit bis zu 18 Kohlenstoffatomen darstellt, gegebenenfalls substituiert durch einen oder mehrere Reste, gewählt aus der Gruppe, die besteht aus: den freien, in Salz überführten, veresterten oder amidierten Carbonsäureresten, dem Hydroxylrest, den Halogenatomen, den Resten NO₂, C≡N, den Resten Alkyl, Alkenyl und Alkinyl, O-Alkyl, O-Alkenyl und O-Alkinyl, S-Alkyl, S-Alkenyl und S-Alkinyl, N-Alkyl, N-Alkenyl und N-Alkinyl, linear, verzweigt oder cyclisch, mit bis zu 12 Kohlenstoffatomen, gegebenenfalls substituiert durch ein oder mehrere Halogenatome, den Rest 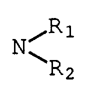 worin R₁ und R₂, gleich oder verschieden, ein Wasserstoffatom oder einen Alkylrest mit bis zu 12 Kohlenstoffatomen, Aryl, heterocyclisches O-Aryl und S-Aryl darstellen, die ein oder mehrere Heteroatome umfassen, gegebenenfalls substituiert durch einen oder mehrere der oben erwähnten Substituenten, sowie ihre Additionssalze mit Säuren.

2. Verbindungen der Formel (I) wie in Anspruch 1 definiert, worin Ar einen Phenylrest darstellt, gegebenenfalls substituiert durch ein Fluoratom, einen Rest CF₃ oder einen Phenylrest.

3. Verbindungen der Formel (I) wie in Anspruch 2 definiert, worin Ar einen Phenylrest darstellt.

4. Die Verbindung der Formel (I) wie in Anspruch 1 definiert, deren Namen folgt:
- 11,12-Dideoxy-3-de-[(2,6-dideoxy-3-C-methyl-3-O-methyl-α-L-ribohexopyranosyl)-oxy]-6-O-methyl-3-oxo-12,11-{oxycarbonyl-[(4-phenyl-3-butenyl)-imino]}-erythromycin und seine Salze mit Säuren.

5. Als Medikamente die Verbindungen der Formel (I) wie in irgendeinem der Ansprüche 1, 2 und 3 definiert, sowie die Additionssalze mit pharmazeutisch akzeptablen Säuren.

6. Als Medikament die Verbindung der Formel (I) wie in Anspruch 4 definiert, sowie ihre Additionssalze mit pharmazeutisch akzeptablen Säuren.

7. Pharmazeutische Zusammensetzungen, enthaltend als Wirkstoff mindestens ein in Anspruch 5 oder 6 definiertes Medikament.

8. Verfahren zur Herstellung der Verbindungen der Formel (I) wie in irgendeinem der Ansprüche 1 bis 4 definiert, dadurch gekennzeichnet, daß man eine Verbindung der Formel (II) 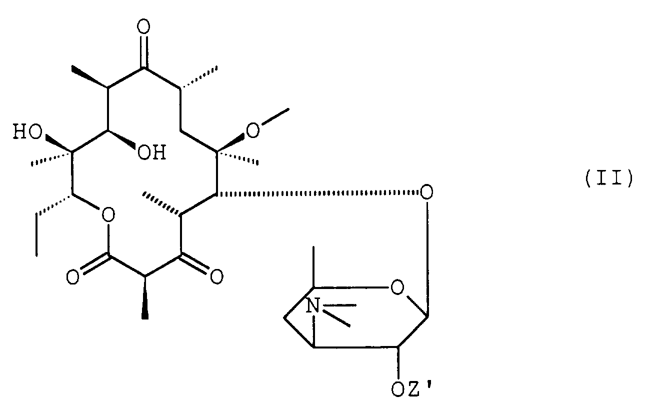 in der Z' den Rest den Rest einer Carbonsäure mit bis zu 18 Kohlenstoffatomen darstellt, der Einwirkung eines Mittels unterzieht, das fähig ist, das Hydroxyl in 11 selektiv zu aktivieren, um die Verbindung der Formel (III) 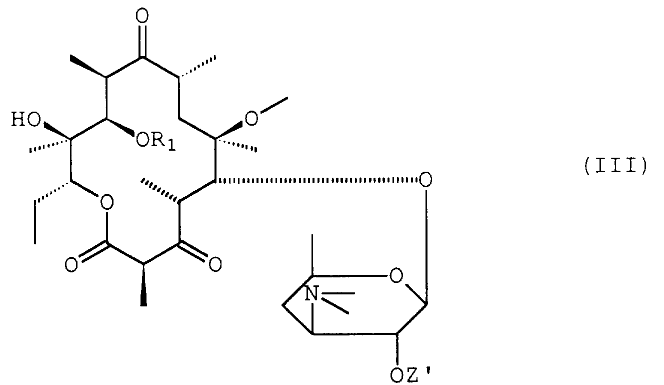 zu erhalten, in der R₁ den Rest der Aktivatorgruppe darstellt, die man der Einwirkung einer Base unterzieht, um die Verbindung der Formel (IV) 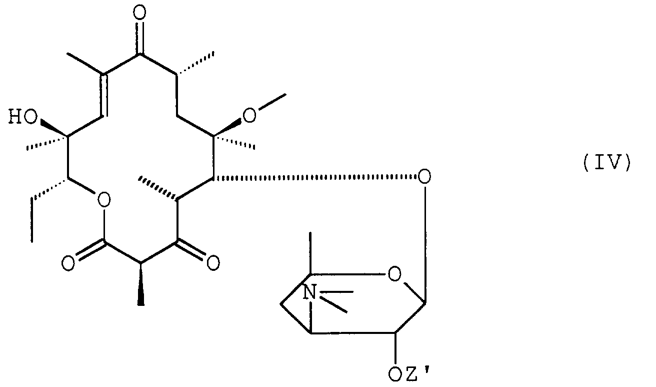 zu erhalten, und man anschließend die Verbindung der Formel (IV)
- entweder der Einwirkung einer Verbindung der Formel (V)
R-N=C=O (V)
unterzieht, in der R wie in Anspruch 1 definiert ist, um die Verbindung der Formel (VI) 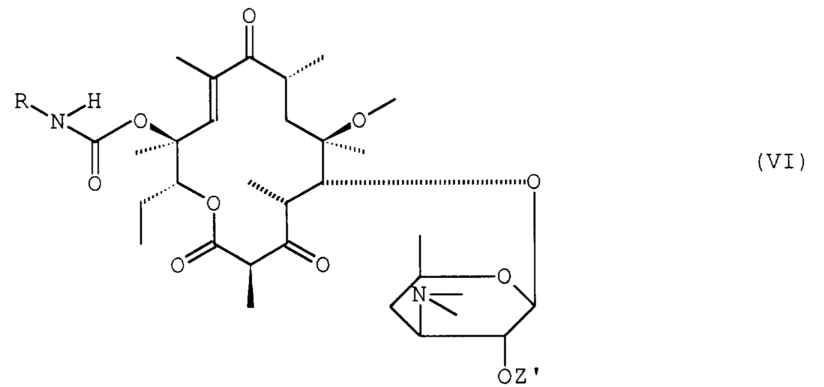 zu erhalten, die sich entweder spontan durch Erhitzen oder dadurch cyclisiert, daß man sie der Einwirkung eines Cyclisierungsmittels unterzieht, um die Verbindung der Formel (IA) 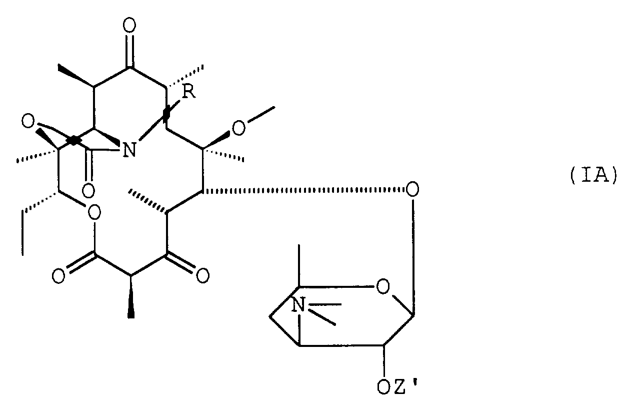 zu erhalten,
- oder der Einwirkung von Carbonyldiimidazol unterzieht, um die Verbindung der Formel (VII) 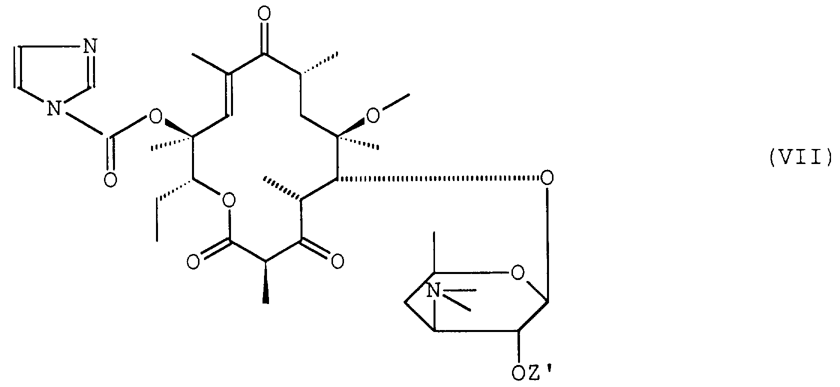 zu erhalten, die man anschließend der Einwirkung einer Verbindung der Formel (VIII)
RNH₂ (VIII)
unterzieht, in der R wie in Anspruch 1 definiert ist, um die wie vorstehend definierte Verbindung der Formel (VI) zu erhalten, die sich entweder spontan durch Erhitzen oder dadurch cyclisiert, daß man sie der Einwirkung eines Cyclisierungsmittels unterzieht, um die entsprechende Verbindung der Formel (IA) zu erhalten, die man der Einwirkung eines Mittels zur Freisetzung der Hydroxyl-Funktion in 2' und/oder gegebenenfalls der Einwirkung einer Säure unterzieht, um das Salz zu bilden.

## Claims

1. The compounds of formula (I): 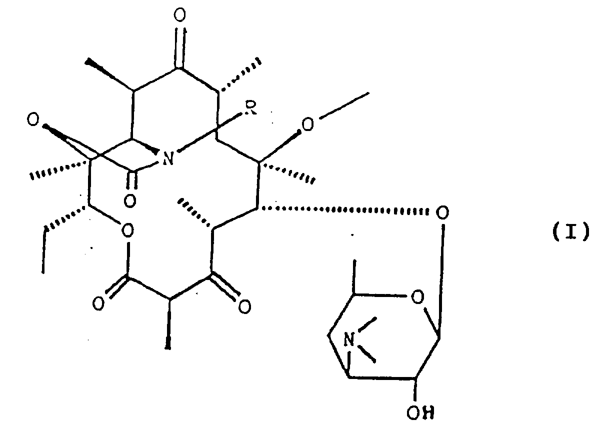 in which R represents a 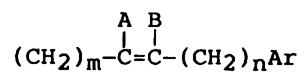 radical, in which m represents the number 1 or 2 and n represents the number 0 or 1,
and either A and B each represent a hydrogen atom, the geometry of the double bond being E or Z or an E + Z mixture, or A and B form a third bond between the carbon atoms to which they are linked,
and Ar represents a carbocyclic aryl radical containing up to 18 carbon atoms optionally substituted by one or more radicals chosen from the group constituted by free, salified, esterified or amidified carboxyl radicals, the hydroxyl radical, halogen atoms, NO₂, C≡N radicals, the following radicals: alkyl, alkenyl and alkynyl, O-alkyl, O-alkenyl and O-alkynyl, S-alkyl, S-alkenyl and S-alkynyl, N-alkyl, N-alkenyl and N-alkynyl, linear, branched or cyclic, containing up to 12 carbon atoms optionally substituted by one or more halogen atoms, the 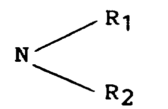 radical, R₁ and R₂, identical or different, representing a hydrogen atom or an alkyl radical containing up to 12 carbon atoms, heterocyclic aryl, O-aryl and S-aryl radicals containing one or more heteroatoms, optionally substituted by one or more of the substituents mentioned above, as well as their addition salts with acids.

2. The compounds of formula (I) as defined in claim 1, in which Ar represents a phenyl radical optionally substituted by a fluorine atom, a CF₃ radical, a phenyl radical.

3. The compounds of formula (I) as defined in claim 2, in which Ar represents a phenyl radical.

4. The compound of formula (I) defined in claim 1 of which the name follows: - 11,12-dideoxy 3-de((2,6-dideoxy 3-C-methyl 3-O-methyl alpha-L-ribohexopyranosyl) oxy) 6-O-methyl 3-oxo 12,11-(oxycarbonyl ((4-phenyl 3-butenyl) imino)) erythromycin, and its salts with acids.

5. As medicaments, the compounds of formula (I) as defined in any one of claims 1, 2 and 3 as well as the addition salts with pharmaceutically acceptable acids.

6. As a medicament, the compound of formula (I) as defined in claim 4, as well as its addition salts with pharmaceutically acceptable acids.

7. The pharmaceutical compositions containing as active ingredient at least one medicament defined in claim 5 or 6.

8. Preparation process for the compounds of formula (I) as defined in any one of claims 1 to 4, characterized in that a compound of formula (II): 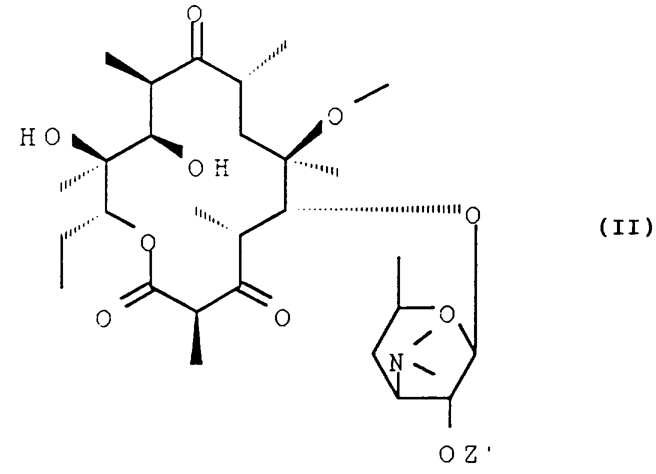 in which Z' represents the remainder of a carboxylic acid containing up to 18 carbon atoms, is subjected to the action of an agent capable of selectively activating the hydroxyl in position 11 in order to obtain the compound of formula (III): 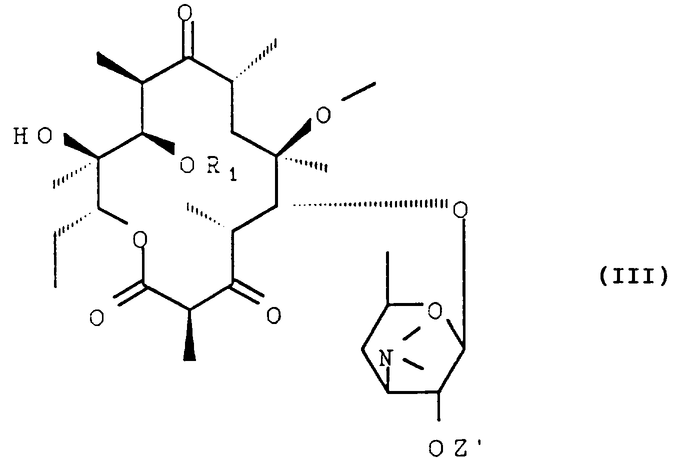 in which R₁ represents the remainder of the activator group, which is subjected to the action of a base, in order to obtain the compound of formula (IV): 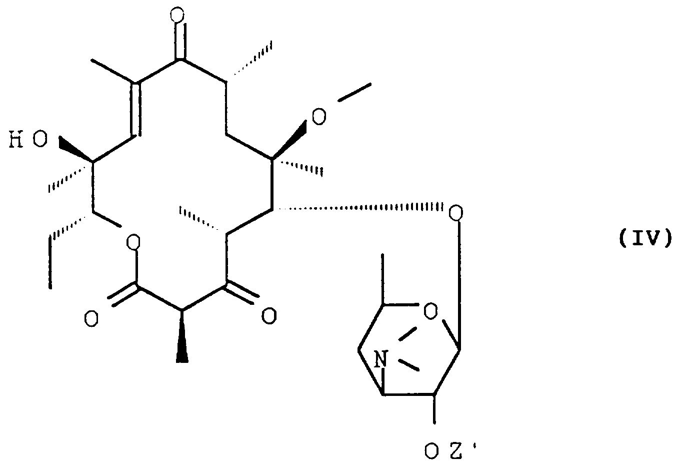 then the compound of formula (IV) is subjected:
- either to the action of a compound of formula (V):
R-N=C=O (V)
in which R is defined as in claim 1, in order to obtain the compound of formula (VI): 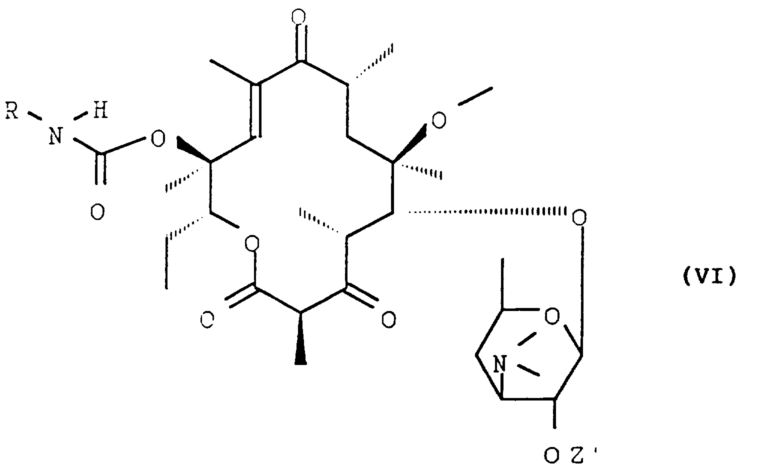 which is cyclized either spontaneously by heating, or which is subjected to the action of a cyclization agent in order to obtain the compound of formula (IA): 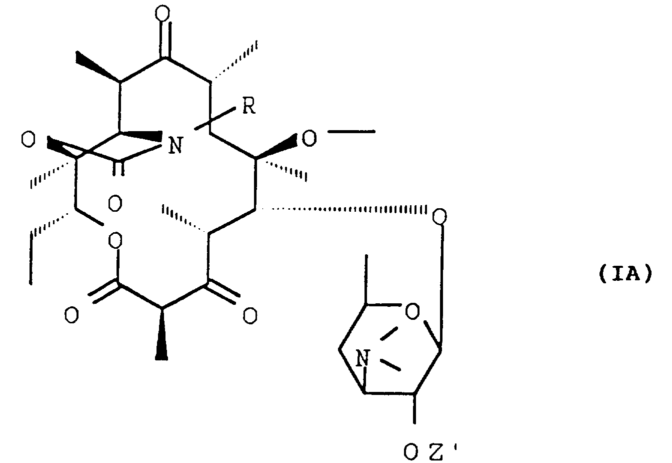
- or to the action of carbonyldiimidazole in order to obtain the compound of formula (VII): 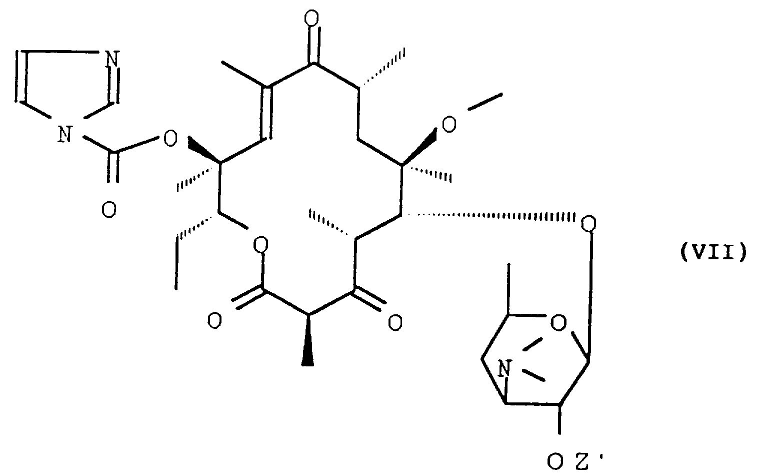 then to the action of a compound of formula (VIII):
RNH₂ (VIII)
in which R is defined as in claim 1, in order to obtain the compound of formula (VI) defined previously which is cyclized spontaneously by heating or which is subjected to the action of a cyclization agent in order to obtain the corresponding compound of formula (IA), then the compound of formula (IA) is subjected to the action of an agent which releases the hydroxyl function in position 2' and/or if appropriate, to the action of an acid in order to form the salt.
